(19) **Europäisches Patentamt / European Patent Office / Office européen des brevets**

(11) **EP 3 238 689 A1**

(12) **EUROPEAN PATENT APPLICATION**
published in accordance with Art. 153(4) EPC

(43) Date of publication:
01.11.2017 Bulletin 2017/44

(21) Application number: 15872568.9

(22) Date of filing: 18.11.2015

(51) Int Cl.:
*A61J 1/00* (2006.01)  *A61K 9/70* (2006.01)
*A61K 31/167* (2006.01)  *A61K 31/27* (2006.01)
*A61K 45/00* (2006.01)  *A61K 47/06* (2006.01)
*A61K 47/14* (2017.01)  *A61K 47/32* (2006.01)
*A61K 47/34* (2017.01)  *A61P 23/02* (2006.01)

(86) International application number:
**PCT/JP2015/082476**

(87) International publication number:
**WO 2016/103999 (30.06.2016 Gazette 2016/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: 26.12.2014  JP 2014265955
30.03.2015  JP 2015069797

(71) Applicant: **Nichiban Co., Ltd.**
**Bunkyo-ku**
**Tokyo**
**112-8663 (JP)**

(72) Inventors:
- **TOHARA, Yukiko**
  **Tokyo 112-8663 (JP)**
- **MATSUO, Kyohei**
  **Tokyo 112-8663 (JP)**
- **OHBA, Ayaka**
  **Tokyo 112-8663 (JP)**
- **NARUSE, Sayaka**
  **Tokyo 112-8663 (JP)**
- **INAGAKI, Kanako**
  **Tokyo 112-8663 (JP)**

(74) Representative: **Schwabe - Sandmair - Marx Patentanwälte Rechtsanwalt Partnerschaft mbB**
**Joseph-Wild-Straße 20**
**81829 München (DE)**

(54) **PACKAGE FOR PATCH AND PACKAGING METHOD**

(57)  To provide a packaging body for a patch exhibiting excellent temporal stability of a drug, a patch product equipped with the patch and a packaging bag, and a method of manufacturing the patch product.

A patch product equipped with a packaging bag formed of a laminate composed of at least three or more layers including a cyclic polyolefin film and a lidocaine- or rivastigmine-containing patch housed in the packaging bag, a method of the patch product, and a packaging body for a patch formed of a laminate including a cyclic polyolefin film.

EP 3 238 689 A1

**Description**

Technical Field

The present invention relates to a packaging body for a patch and a method of packing a patch, specifically, it relates to a packaging body for a patch and a method of manufacturing a packaging body for a patch, which can suppress a decrease in drug content in the patch with the lapse of time by providing a layer of a cyclic polyolefin (hereinafter referred to as the COC in some cases) film to a packaging material particularly when a patch containing lidocaine of a local anesthetic or a patch containing rivastigmine of an antidementia drug is packed in a packaging body.

Background Art

Several packaging materials have been proposed in order to suppress the decomposition and the like of the drug contained in a patch and to stably store the patch for a long period of time.

For example, Patent Literature 1 proposes a packaging bag for patch in which a hygroscopic member layer, a water permeable member layer, and a shielding member layer are laminated for the purpose of providing a packaging bag for patch which can sufficiently decrease the influence of moisture on the drug in a patch so as to stably store the patch for a long period of time and also exhibits excellent economic efficiency and handling property.

Patent Literature 2 proposes a packaging structure obtained by hermetically packing the entire patch in which a pressure sensitive adhesive layer containing a fatty acid ester and/or a glycerin ester as an organic liquid component having a melting point of 25°C or lower at from 40 weight% to 70 weight% in a compatible state is provided on one surface of a support, in which the packaging material is a laminate and the inner surface thereof is formed of an ethylene/vinyl alcohol copolymer film or an acrylonitrile/methyl acrylate copolymer film as a plastic material having a solubility parameter of 9 or more.

Patent Literature 3 proposes a laminated packaging bag housing a transdermal absorbent that permeates a medicinal ingredient via the skin, in which at least one layer of the heat-sealed layer includes an ethylene-vinyl alcohol copolymer (EVOH) layer and has a chuck-like opening composed of polyethylene.

Meanwhile, various proposals on the blending of plaster have been made with regard to the stability of a patch containing a local anesthetic, for example, lidocaine.

For example, Patent Literature 4 describes that it is preferable to adjust the pH of a hydrous patch to from 5 to 9 in consideration of the stability of lidocaine.

Patent Literature 5 describes an example in which an oxidation inhibitor (BHT) is blended in a transdermal absorption type preparation in order to improve storage stability thereof.

Patent Literature 6 describes that an acrylic pressure sensitive adhesive exhibits high polymerizability, a high cohesive force, and sufficient adhesive property and can stably retain a large amount of local anesthetic.

Patent Literature 7 describes that a stable base layer can be formed by blending a vinyl acetate resin.

In addition, rivastigmine is one of acetylcholinesterase inhibitors and used as a therapeutic agent for Alzheimer type dementia (antidementia drug), and in recent years, the transdermal administration thereof, namely, administration thereof using a patch has also been proposed.

It has been pointed out that rivastigmine is considered to be relatively susceptible to oxidation and it is concerned that the decomposition products thereof increase with the lapse of time. Hence, a technique to blend an antioxidant in a patch containing rivastigmine has been proposed (Patent Literature 8, Patent Literature 9, and the like). In Patent Literature 5, a preparation in which a silicone pressure sensitive adhesive is pasted to a storage layer containing rivastigmine has been reported, but the silicone pressure sensitive adhesive has a problem that the cost of the pressure sensitive adhesive itself is high and the cost of the release liner is also high since it is required to be subjected to special processing.

In addition, acrylic pressure sensitive adhesives containing a carboxyl group are used in patches proposed in Patent Literature 8, Patent Literature 9, and the like, but this use considerably affects the oxidation of rivastigmine, and it has been thus reported that a patch exhibiting excellent temporal stability of rivastigmine can be obtained by using a pressure sensitive adhesive which does not have a carboxyl group or a hydroxyl group as an acrylic pressure sensitive adhesive (Patent Literature 10).

Furthermore, a patch (Patent Literature 11) using an acryl-rubber hybrid adhesive having a hydroxyl group is proposed from the viewpoint of improving the skin permeability of rivastigmine and decreasing the skin irritation by the pressure sensitive adhesive layer, and a patch (Patent Literature 12) in which a nonvolatile substance such as citric acid is contained in an acrylic pressure sensitive adhesive layer having a hydroxyl group and the like has been proposed for the purpose of decreasing the loss of the active substance (drug) due to the volatilization in the manufacturing process.

However, the stability of rivastigmine is not discussed in these literatures.

Citation List

Patent Literature

Patent Literature 1: JP 2001-9985 A
Patent Literature 2: JP 3575616 B1
Patent Literature 3: JP 2010-280403 A
Patent Literature 4: JP 3115625 B1
Patent Literature 5: WO 2011/027786 A
Patent Literature 6: JP 6-145051 A
Patent Literature 7: JP 2004-292379 A
Patent Literature 8: JP 2002-500178 W
Patent Literature 9: JP 2009-517468 W
Patent Literature 10: WO 2011/076621 A
Patent Literature 11: US 2011/0066120
Patent Literature 12: EP 2172194 B

Summary of Invention

Technical Problem

As described above, various investigations have been conducted on the techniques capable of eliminating temporal instability due to the influence of moisture and oxygen and of stably storing the patch for a long period of time. However, sufficient investigations have not been necessarily conducted from the viewpoint of eliminating the temporal instability of the local anesthetic, particularly lidocaine.
In addition, in the patch containing rivastigmine, it has been proposed to blend an antioxidant or to use a pressure sensitive adhesive which does not have a carboxyl group or a hydroxyl group from the viewpoint of eliminating temporal instability of rivastigmine due to the oxidation, but sufficient investigations have not been necessarily conducted on the packaging container itself which houses the rivastigmine-containing patch.

Solution to Problem

The present inventors have conducted intensive studies to solve the above problems, and they have investigated the configurations of packaging materials to house these patches in order to eliminate temporal instability of a drug of which the stability is susceptible to oxidation, volatilization, and adsorption, particularly a patch containing lidocaine of a local anesthetic and rivastigmine of an antidementia drug. As a result, the present inventors have found out that the above problem can be solved by adopting a laminate composed of at least three or more layers including a cyclic polyolefin film as a packaging bag which houses a patch containing a drug (lidocaine or rivastigmine) of which the stability is susceptible to oxidation, volatilization, and adsorption, thereby completing the present invention.
The present invention provides the following patch product as an aspect.

(1) A patch product comprising:

a packaging bag; and
a patch which contains a drug and is housed in the packaging bag, wherein
the packaging bag is formed of a laminate composed of at least three or more layers including a cyclic polyolefin film, wherein
the laminate has a laminate configuration having a heat sealable cyclic polyolefin film as an innermost layer of the packaging bag, an oxygen blocking layer as a layer on an outer side of the cyclic polyolefin film, and an outer layer as a layer on an outer side of the oxygen blocking layer disposed, respectively.

In particular, the present invention provides the following patch product.
(2) The patch product according to (1), wherein
the patch includes:

a support, drug-containing layer which contains a drug and is provided on at least one surface of the support, and a pressure sensitive adhesive layer provided on the drug-containing layer, or

a support, a pressure sensitive adhesive layer provided on at least one surface of the support, and a drug-containing layer which contains a drug and is provided on the pressure sensitive adhesive layer, or

a support and a drug-containing pressure sensitive adhesive layer provided on at least one surface of the support.

(3) The patch product according to (2), wherein
the support is a fabric selected from the group consisting of a nonwoven fabric, a woven fabric, and a knitted fabric.
(4) The patch product according to (2) or (3), wherein
the support includes at least one kind of material selected from the group consisting of polyester and polyolefin.
(5) The patch product according to any one of (2) to (4), wherein the drug is lidocaine.
(6) The patch product according to any one of (2) to (4), wherein the drug is a drug which volatilizes at room temperature.
(7) The patch product according to (6), wherein the drug is rivastigmine.
(8) The patch product according to (5), wherein
a content of a drug contained in a drug-containing layer is from 0.1 mass% to 50 mass% based on a total mass of the drug-containing layer and a pressure sensitive adhesive layer, or
a content of a drug contained in a drug-containing pressure sensitive adhesive layer is from 0.1 mass% to 50 mass% based on a total mass of the drug-containing pressure sensitive adhesive layer in the patch.
(9) The patch product according to (7), wherein
a content of a drug contained in a drug-containing layer is from 10 mass% to 40 mass% based on a total mass of the drug-containing layer and a pressure sensitive adhesive layer, or
a content of a drug contained in a drug-containing pressure sensitive adhesive layer is from 10 mass% to 40 mass% based on a total mass of the drug-containing pressure sensitive adhesive layer in the patch.
(10) The patch product according to (9), wherein
the pressure sensitive adhesive layer or the drug-containing pressure sensitive adhesive layer contains an acrylic pressure sensitive adhesive.
(11) The patch product according to any one of (1) to (10), wherein a residual oxygen concentration in the packaging bag is 10 vol% or less.
 In addition, the present invention provides the following packaging body as another aspect.
(12) A packaging body for a patch containing lidocaine, wherein
the packaging body is formed of a laminate composed of at least three or more layers including a cyclic polyolefin film, wherein
the laminate has a laminate configuration having a heat sealable cyclic polyolefin film as an innermost layer of the packaging body, an oxygen blocking layer as a layer on an outer side of the cyclic polyolefin film, and an outer layer as a layer on an outer side of the oxygen blocking layer disposed, respectively.
(13) A packaging body for a patch containing rivastigmine, wherein
the packaging body is formed of a laminate composed of at least three or more layers including a cyclic polyolefin film, wherein
the laminate has a laminate configuration having a heat sealable cyclic polyolefin film as an innermost layer of the packaging body, an oxygen blocking layer as a layer on an outer side of the cyclic polyolefin film, and an outer layer as a layer on an outer side of the oxygen blocking layer disposed, respectively.
(14) The packaging body according to (12) or (13), wherein a residual oxygen concentration in the packaging body is 10 vol% or less.
Furthermore, the present invention provides a method of manufacturing the patch product described above as an aspect.
(15) A method of manufacturing the patch product according to any one of (1) to (11), the method comprising:

a step of preparing a laminate which includes a film of cyclic polyolefin and has a heat sealable layer as a surface layer;
a step of fabricating a bag-like article provided with an opening by superposing the two laminates such that the heat sealable layers are face each other and heat-sealing the superposed laminates;
a step of loading a patch in the bag-like article through the opening; and
a step of fabricating a packaging bag with patch by sealing the opening of the bag-like article by heat sealing.

(16) The manufacturing method according to (15), further comprising:

a step of purging an interior of the bag-like article with nitrogen and/or loading an oxygen absorbent in the bag-like article through the opening; and

a step of discharging a gas inside the bag-like article to an outside to an extent that the two laminates tightly sandwich the patch by roll pass, pressing, or suction degassing

before the step of fabricating a packaging bag with patch by sealing the opening of the bag-like article by heat sealing.

(17) A method of manufacturing the patch product according to any one of claims 1 to 11, the method comprising:

a step of preparing a laminate which includes a film of cyclic polyolefin and has a heat sealable layer as a surface layer;

a step of disposing a patch on the heat sealable layer of one laminate;

a step of disposing the other laminate on the one laminate such that the heat sealable layers of the two laminates face each other via the patch;

a step of forming an opening by heat-sealing a greater part of the two heat sealable layers facing each other but not heat-sealing a part of the two heat sealable layers; and

a step of fabricating a packaging bag with patch by heat-sealing the opening.

(18) The manufacturing method according to claim 17, further comprising the following steps before fabricating a packaging bag with patch by heat-sealing the opening:

a step of purging an atmosphere in a vicinity of the patch to be formed by the two laminates with nitrogen and/or loading an oxygen absorbent in a vicinity of the patch; and

a step of discharging a gas in a vicinity of the patch to an outside through the opening to an extent that the two laminates tightly sandwich the patch by roll pass, pressing, or suction degassing.

Advantageous Effects of Invention

The present invention exerts an effect that it is possible to suppress adsorption of lidocaine or rivastigmine to a packaging bag and to suppress a decrease in the drug content in the plaster of a patch with the lapse of time by a configuration in which the packaging bag which houses a patch containing lidocaine (hereinafter, referred to as the lidocaine-containing patch) as a drug or a patch containing rivastigmine (hereinafter, referred to as the rivastigmine-containing patch) as a drug is formed of a laminate composed of at least three or more layers including a cyclic polyolefin film.

Description of Embodiments

<Patch Product>

The present invention relates to a patch product equipped with a packaging bag and a patch which contains a drug and is housed in the packaging bag, particularly a patch containing a drug (lidocaine or rivastigmine) of which the stability is susceptible to oxidation, volatilization, and adsorption.

The drug contained in a patch to be a target of the patch product of the present invention, for example, lidocaine is considered to be easily adsorbed and rivastigmine is considered to be relatively susceptible to oxidation and volatilization. Hence, it is preferable that the patches containing these drugs are housed in a packaging bag fabricated with a highly non-adsorptive, sealable, and light blocking packaging material and stored until immediately before being used.

In the present invention, a cyclic polyolefin (hereinafter, also referred to as "COC") film capable of suppressing the adsorption of lidocaine or rivastigmine is adopted as an essential constituent in the laminate constituting the packaging material to enclose the patch.

<Packaging Bag>

The packaging bag to be used in the present invention is formed of a laminate composed of at least three or more layers including a cyclic polyolefin film (hereinafter, also referred to as the COC film).

The laminate has a laminate configuration in which a heat sealable cyclic polyolefin film as an innermost layer of the packaging bag, an oxygen blocking as a layer on the outer side of the cyclic polyolefin film, and an outer layer as a layer on the outer side of the oxygen blocking layer are disposed, respectively.

[Cyclic Polyolefin (COC) Film]

The innermost layer of the packaging bag is a heat sealable layer, namely, a heat sealable cyclic polyolefin (COC) film. A cyclic polyolefin is a polymer having a cyclic olefin structure and also referred to as a cycloolefin polymer or the like.

The cyclic polyolefin film exhibits high barrier property and also excellent non-adsorptive property so as to be preferable as a material for a packaging container to enclose a rivastigmine-containing patch.

As to be described in detail below, the cyclic polyolefin film can be formed of a cyclic olefin polymer (also referred to as COP) composed only of a cyclic olefin component, a cyclic olefin copolymer that is a copolymer of a cyclic olefin component with an olefin component such as ethylene, or a blended polymer obtained by blending a cyclic olefin polymer or a cyclic olefin copolymer with another polyolefin resin.

Examples of the cyclic olefin component in the COC film may include cyclohexane or a derivative thereof, cycloheptene or a derivative thereof, cyclooctene or a derivative thereof, cyclononene or a derivative thereof, cyclodecene or a derivative thereof, bicyclo[2.2.1] hept-2-ene or a derivative thereof, tetracyclo[$4.4.0.1^{2,5}.1^{7,10}$]-3-dodecene or a derivative thereof, hexacyclo[$6.6.1.1^{3,6}.1^{10,13}.0^{2,7}.0^{9,14}$]-4-heptadecene or a derivative thereof, octacyclo[$8.8.0.1^{2,9}.1^{4,71}1^{,10}.1^{13,16}.0^{3,8}.0^{12,17}$]-5-docosene or a derivative thereof, pentacyclo[$6.6.1.1^{3,6}.0^{2,7}.0^{9,14}$]-4-hexadecene or a derivative thereof, pentacyclo[$6.5.1.1^{3,6}.0^{2,7}.0^{9,13}$]-4-pentadecene or a derivative thereof, heptacyclo[$8.7.0.1^{2,9}.1^{4,7}.1^{11,17}.0^{3,8}.0^{12,16}$]-5-eicosene or a derivative thereof, heptacyclo[$8.8.0.1^{2,9}.1^{4,7}.1^{11,16}.0^{3,8}.0^{12,17}$]-5-heneicosene or a derivative thereof, tricyclo[$4.4.0.1^{2,5}$]-3-undecene or a derivative thereof, tricyclo[$4.3.0.1^{2,5}$]-3-decene or a derivative thereof, tricyclo[$4.3.0.1^{2,5}$]-3,7-decadiene or a derivative thereof, pentacyclo[$6.5.1.1^{3,6}.0^{2,7}.0^{9,13}$]-4,10-pentadecadiene or a derivative thereof, pentacyclo[$4.7.0.1^{2,5}.0^{8,13}.1^{9,12}$]-3-pentadecene or a derivative thereof, heptacyclo[$7.8.0.1^{3,6}.0^{2,7}.1^{10,17}.0^{11,16}.1^{12,15}$]-4-eicosene or a derivative thereof, and nonacyclo[$9.10.1.1^{4,7}.0^{3,8}.0^{2,10}.0^{12,21}.1^{13,20}.0^{14,19}.1^{15,19}$]-5-pentacecone or a derivative thereof. These components may be used singly or in mixture of two or more thereof

Furthermore, examples of the cyclic olefin component may include ring-opened products and hydrogenated products thereof such as bicyclo[2.2.1]hept-2-ene or a derivative thereof, tetracyclo[$4.4.0.1^{2,5}.1^{7,10}$]-3-dodecene or a derivative thereof, hexacyclo[$6.6.1.1^{3,6}.1^{10,13}.0^{2,7}.0^{9,14}$]-4-heptadecene or a derivative thereof, octacyclo[$8.8.0.1^{2,9}.1^{4,7}.1^{11,10}.1^{13,16}.0^{3,8}.0^{12,17}$]-5-docosene or a derivative thereof, pentacyclo[$6.6.1.1^{3,6}.0^{2,7}.0^{9,14}$]-4-hexadecene or a derivative thereof, pentacyclo[$6.5.1.1^{3,6}.0^{2,7}.0^{9,13}$]-4-pentadecene or a derivative thereof, heptacyclo[$8.7.0.1^{2,9}.1^{4,7}.1^{11,17}.0^{3,8}.0^{12,16}$]-5-eicosene or a derivative thereof, heptacyclo[$8.8.0.1^{2,9}.1^{4,7}.1^{11,16}.0^{3,8}.0^{12,17}$]-5-heneicosene or a derivative thereof, tricyclo[$4.4.0.1^{2,5}$]-3-undecene or a derivative thereof, tricyclo[$4.3.0.1^{2,5}$]-3-decene or a derivative thereof, tricyclo[$4.3.0.1^{2,5}$]-3,7-decadiene or a derivative thereof, pentacyclo[$6.5.1.1^{3,6}.0^{2,7}.0^{9,13}$]-4,10-pentadecadiene or a derivative thereof, pentacyclo[$4.7.0.1^{2,5}.0^{8,13}.1^{9,12}$]-3-pentadecene or a derivative thereof, heptacyclo[$7.8.0.1^{3,6}.0^{2,7}.1^{10,17}.0^{11,16}.1^{12,15}$]-4-eicosene or a derivative thereof, and nonacyclo[$9.10.1.1^{4,7}.0^{3,8}.0^{2,10}.0^{12,21}.1^{13,20}.0^{14,19}.1^{15,19}$]-5-pentacecone or a derivative thereof These components may be used singly or in mixture of two or more thereof

In addition, examples of the olefin component may include ethylene, propylene, 1-butene, 1-pentene, 4-methyl-pentene, 3-methyl-pentene, 1-hexene, 1-heptene, 1-octene, 1-nonene, and 1-decene. These components may be used singly or in mixture of two or more thereof

In addition, in the cyclic olefin copolymer, the structural unit derived from the olefin component such as ethylene is suitably in a range of from 40 mol% to 95 mol% and the structural unit derived from the cyclic olefin component is usually suitably in a range of from 5 mol% to 60 mol%.

Furthermore, as the COC polymer, a ring-opened (co) polymer obtained by ring-opening (co) polymerization of COC and a hydrogenated product thereof can be used. Examples thereof may include a single ring-opened polymer or a ring-opened copolymer of two or more components and hydrogenated products thereof such as bicyclo[2.2.1]hept-2-ene or a derivative thereof, tetracyclo[$4.4.0.1^{2,5}.1^{7,10}$]-3-dodecene or a derivative thereof, hexacyclo[$6.6.1.1^{3,6}.1^{10,13}.0^{2,7}.0^{9,14}$]-4-heptadecene or a derivative thereof, octacyclo[$8.8.0.1^{2,9}.1^{4,7}.1^{11,10}.1^{13,16}.0^{3,8}.0^{12,17}$]-5-docosene or a derivative thereof, pentacyclo[$6.6.1.1^{3,6}.0^{2,7}.0^{9,14}$]-4-hexadecene or a derivative thereof, pentacyclo[$6.5.1.1^{3,6}.0^{2,7}.0^{9,13}$]-4-pentadecene or a derivative thereof, heptacyclo[$8.7.0.1^{2,9}.1^{4,7}.1^{11,17}.0^{3,8}.0^{12,16}$]-5-eicosene or a derivative thereof, heptacyclo[$8.8.0.1^{2,9}.1^{4,7}.1^{11,16}.0^{3,8}.0^{12,17}$]-5-heneicosene or a derivative thereof, tricyclo[$4.4.0.1^{2,5}$]-3-undecene or a derivative thereof, tricyclo[$4.3.0.1^{2,5}$]-3-decene or a derivative thereof, tricyclo[$4.3.0.1^{2,5}$]-3,7-decadiene or a derivative thereof, pentacyclo[$6.5.1.1^{3,6}.0^{2,7}.0^{9,13}$]-4,10-pentadecadiene or a derivative thereof, pentacyclo[$4.7.0.1^{2,5}.0^{8,13}.1^{9,12}$]-3-pentadecene or a derivative thereof, heptacyclo[$7.8.0.1^{3,6}.0^{2,7}.1^{10,17}.0^{11,16}.1^{12,15}$]-4-eicosene or a derivative thereof, and nonacyclo[$9.10.1.1^{4,7}.0^{3,8}.0^{2,10}.0^{12,21}.1^{13,20}.0^{14,19}.1^{15,19}$]-5-pentacecone or a derivative thereof

As the COC polymer, for example, TOPAS (registered trademark, manufactured by Polyplastics Co., Ltd.), APEL (registered trademark, manufactured by Mitsui Chemicals, Inc.), and ARTON (registered trademark, manufactured by JSR Corporation) of a commercially available product can be used.

In addition, the COC film can be a film blended with an olefin resin such as a linear low density polyethylene (LLDPE) resin, a high density polyethylene (HDPE) resin, or a polypropylene (PP) resin in consideration of adhesive property (heat sealability), the amount of low molecular weight substance eluted, and the like. The blending rate (mass ratio) of the resin other than COC constituting the COC film is preferably from 3 mass% to 50 mass% and particularly preferably from 5 mass% to 10 mass%. In addition, the density of linear low density polyethylene is from 0.935 to 0.950, the density of high density polyethylene is from 0.940 to 0.975, and polypropylene may be either a homo or block type, but a homo type is preferred.

In addition, the COC film may further contain one kind or two or more kinds of additives such as an oxidation inhibitor, an ultraviolet absorber, a light stabilizer, an antistatic agent, an antiblocking agent, a lubricant (fatty acid amide or the like), a flame retardant, an inorganic or organic filler, a crosslinking agent, a coloring agent such as a dye or a pigment, and a modifying resin if necessary.

When the blending rate of the olefin-based resin is less than 3 mass% with respect to the entire mixture, proper fluidity cannot be imparted to the cyclic polyolefin-based resin and this causes the generation of gel lump in some cases.

On the other hand, when the blending rate of the olefin-based resin is more than 50 mass%, it is concerned that the non-adsorptive property of cyclic polyolefin is impaired and also the transparency deteriorates in some cases although the effect of the present invention is not directly affected.

The COC film has a minimum thickness capable of fabricating a hermetically sealed packaging bag by heat sealing, and the thickness is from 10 μm to 50 μm, and for example, preferably 30 μm.

As the COC film, for example, ZeonorFilm (registered trademark, manufactured by ZEON CORPORATION) of a commercially available product can be used.

[Oxygen Blocking Layer]

The oxygen blocking layer to be disposed on the outer side of the COC film may be any material as long as it can block the incorporation of oxygen from the outside of the packaging bag. For example, a vapor deposited layer formed by aluminum vapor deposition, silica vapor deposition, alumina vapor deposition, and silica alumina binary vapor deposition, and the like can be adopted in addition to a soft metal foil such as an aluminum foil.

[Outer Layer]

As the outer layer to be disposed on the outer side of the oxygen blocking layer, it is preferable to adopt one having a mechanical strength and dimensional stability. Examples thereof may include a resin film such as a polyester-based resin film such as polyethylene terephthalate (PET), polyethylene naphthalate (PEN), or polylactic acid (PLA); a polyolefin-based resin film such as polypropylene; a polystyrene-based resin film; a polyamide-based resin film such as 6-nylon or poly-p-xylylene adipamide (MXD6 nylon); a polycarbonate-based resin film; a polyacrylonitrile-based resin film; and a polyimide-based resin film. Furthermore, a paper layer such as paper or synthetic paper may be adopted as the outer layer.

In addition, as the outer layer, a multilayered body (for example, nylon 6/MXD/nylon 6, or nylon 6/cyclic polyolefin/nylon 6) or a mixture of the above resin films may be used.

The outer layer has a function to maintain the strength of the laminate itself, a function as a base material to support the oxygen blocking layer, and a function to counteract the external physical stimulus or chemical stimulus (pressure, friction, and the like) when it is formed into a packaging bag, and it may be in the form of one outer layer having all of these functions or in the form of a plurality of outer layers in which these functions are shared among the plurality of layers.

Incidentally, in the present invention, the "outer layer" means a layer positioned on the outer side of the oxygen blocking layer, and it may be a layer having the positional relationship. In a case in which there are a plurality of outer layers, there may be an outer layer on the most outer side (namely, the outermost layer) and an outer layer positioned between the outer layer on the most outer side and the oxygen blocking layer.

[Laminate]

The packaging bag to be used in the present invention is formed of a laminate composed of at least three or more layers including a cyclic polyolefin (COC) film. The laminate may be one in which a heat sealable COC film as the innermost layer of the packaging bag, an oxygen blocking layer as a layer on the outer side of the COC film, and an outer layer as a layer on the outer side of the oxygen blocking layer are disposed. The packaging bag may have an aspect in which a plurality of the COC film, the oxygen blocking layer, and the outer layer are disposed. As the laminate configuration of the laminate, it is possible to take various aspects, for example, COC film/oxygen blocking layer/outer layer, COC film/oxygen blocking layer/oxygen blocking layer/outer layer, COC film/oxygen blocking layer/outer layer/oxygen blocking layer/outer layer, COC film/COC film/oxygen blocking layer/outer layer, and COC film/oxygen blocking layer/COC film/oxygen blocking layer/outer layer.

In addition, the laminate may have a laminate configuration which further includes layers other than the COC film, the oxygen blocking layer, and the outer layer if necessary.

Examples of the other layers may include a polyolefin-based resin film such as a polyethylene film, a polypropylene film, or a polymethylpentene film; a vinyl-based resin film such as a polyvinyl chloride film, a polyvinylidene chloride film, a polyvinyl alcohol film, a polystyrene film, a polyacrylonitrile film, or an ionomer film; a polyester-based resin film such as a polyethylene terephthalate film; a polyamide-based resin film such as a nylon film; a cellulose-based resin films such as cellophane; a polycarbonate-based resin film; and a laminated film thereof as a packaging material which has high sealing performance and light blocking property and can be used as a layer constituent of a packaging bag, and a laminated film of a soft aluminum foil (hereinafter, abbreviated as AL in some cases) with the resin films and the laminated film thereof described above and a pigment-added resin film obtained by adding a black pigment or the like to the resin films described above as the packaging material which enhances the light blocking property in addition to the sealing performance. In the other layers, these resin films, laminated films, and the like can be used as a laminate in various combinations.

In addition, as the other layers, it is also possible to dispose a film or sheet including a layer containing an oxygen adsorbent. The oxygen absorbent is not particularly limited, but examples thereof may include a mixture composed of an electrolyte, a reducing inorganic salt, a polyphenol, a reducing saccharide, a reducing agent, an unsaturated fatty acid compound, an unsaturated organic compound, a thermoplastic polymer, a composition containing an oxygen absorption promoting substance, or a mixture thereof in addition to an inorganic oxide. Examples of the packaging material containing an oxygen absorbent may include OxyGuard (registered trademark) (manufactured by Toyo Seikan Co., Ltd.) and OxyCatch (registered trademark) (manufactured by KYODO PRINTING CO., LTD.).

An example of a specific laminate configuration of a preferred laminate to be used in the packaging bag of the present invention is a configuration having COC/AL/polyester laminated in this order from the innermost layer of the packaging bag.

The laminate can be manufactured by dry lamination, extrusion lamination, or coextrusion lamination, and the thickness of the laminate can be set to about 10 $\mu$m to 100 $\mu$m. The respective layers are directly laminated or laminated via an adhesive or the like. In the case of joining the layers with an adhesive, it is desirable to use an adhesive to which the drug is hardly adsorbed. For example, an isocyanate such as TDI or MDI is used as the adhesive, or a polyurethane adhesive blended with a polyol or the like can be used. The packaging body is formed to be one size larger than the lidocaine-containing patch or rivastigmine-containing patch. Preferably, the packaging body has a size to be from 1.5-fold to 10-fold the patch. For example, the packaging body can be 13.5 cm long $\times$ 17.5 cm wide when the patch is 10 cm long $\times$ 14 cm wide. In addition, for example, the packaging body can be 6 cm long $\times$ 6 cm wide when the patch is 3 cm long $\times$ 3 cm wide. In addition, it is also possible to cut the corners round to attach R in the same manner as the patch.

In the patch product of the present invention, it is possible to set the residual oxygen concentration inside the packaging bag formed of the laminate to, for example, 10 vol% or less. In particular, rivastigmine is susceptible to oxidation, and the generation of decomposition products can be thus suppressed by adjusting the residual oxygen concentration.

As the specific method of setting the residual oxygen concentration inside the packaging bag to 10 vol% or less, it is possible to adopt (I) a method in which the atmosphere in the packaging bag is purged and filled with nitrogen by using a vacuum gas packaging machine or the like, (II) a method in which a film or sheet including a layer containing an oxygen adsorbent is used as a layer constituting the laminate of the packaging bag, and (III) a method in which an oxygen adsorbent is allowed to exist inside the packaging bag in the form of being separately enclosed.

Among them, (I) it is preferable to set the residual oxygen concentration inside the packaging bag to 10 vol% or less by purging and filling the atmosphere inside the packaging bag with nitrogen by using a vacuum gas packing machine or the like from the viewpoint of stability of the drug (for example, rivastigmine).

Specifically, the patch product of the present invention is stored by housing a patch in a packaging bag manufactured from the laminate described above, preferably setting the residual oxygen concentration in the packaging bag to 10 vol% or less, and then sealing the packaging bag by a known method such as heat sealing. The heat-sealed width at this time can be appropriately determined, but it is preferably about from 2 mm to 20 mm.

Incidentally, in the laminate constituting the packaging bag of the present invention, in the case of adopting a plastic laminate film material containing aluminum, it is preferable to provide a V-shaped notch or an I-shaped notch at the edge of the packaging bag since the plastic laminate film has a high tear strength and it is thus difficult to open the packaging bag by the hand.

<Packaging Body>

The present invention targets a packaging body for a patch containing a drug, in particular, a patch containing lidocaine and also a packaging body for a patch containing rivastigmine. In other words, the present invention targets a packaging body formed of a laminate composed of at least three or more layers including a cyclic polyolefin film. The laminate has a laminate configuration in which a heat sealable cyclic polyolefin film as an innermost layer of the packaging body, an oxygen blocking layer as a layer on the outer side of the cyclic polyolefin film, and an outer layer as a layer on the outer side of the oxygen blocking layer are disposed, respectively. Moreover, it is desirable that the residual oxygen concentration inside the packaging body is 10 vol% or less.

Here, a laminate having the same laminate configuration as the laminate to be used in the packaging bag of the patch product described above can be adopted as the laminate constituting the packaging body. In addition, as a preferred aspect, the method mentioned in (I) to (III) above can be adopted as the means for setting the residual oxygen concentration to 10 vol% or less as well.

<Method of Manufacturing Patch Product>

The method of manufacturing the patch product of the present invention is not particularly limited, but examples thereof may roughly include the following two processes. Incidentally, the laminate to be used in [1] and [2] is a laminate having the same laminate configuration as the laminate to be used in the packaging bag of the patch product described above.

[1] A step of preparing a laminate which includes a film of cyclic polyolefin and has a heat sealable layer as the surface layer,
a step of fabricating a bag-like article provided with an opening by superposing the two laminates such that the heat sealable layers thereof are face each other and heat-sealing the superposed laminates,
a step of loading a lidocaine-containing patch or a rivastigmine-containing patch in the bag-like article through the opening,
a step of purging the interior of the bag-like article with nitrogen and/or loading an oxygen absorbent in the bag-like article through the opening (arbitrary step),
a step of discharging the gas (oxygen, nitrogen, and the like) inside the bag-like article to the outside to an extent that the two laminates tightly sandwich the patch by roll pass, pressing, or suction degassing (arbitrary step), and
a step of fabricating a packaging bag with patch by sealing the opening of the bag-like article by heat sealing.
[2] A step of preparing a laminate which includes a film of cyclic polyolefin and has a heat sealable layer as the surface layer,
a step of disposing a lidocaine-containing patch or a rivastigmine-containing patch on the heat sealable layer of one laminate,
a step of disposing the other laminate on the one laminate such that the heat sealable layers thereof face each other via the patch,
a step of forming an opening by heat-sealing a greater part of the two heat sealable layers facing each other but not heat-sealing a part of the two heat sealable layers,
a step of purging the atmosphere in the vicinity of the patch to be formed by the two laminates with nitrogen and/or loading an oxygen absorbent in the vicinity of the patch (arbitrary step),
a step of discharging the gas (oxygen, nitrogen, and the like) in the vicinity of the patch to the outside through the opening to an extent that the two laminates tightly sandwich the patch by roll pass, pressing, or suction degassing (arbitrary step), and
a step of fabricating a packaging bag with patch by heat-sealing the opening.

In order to purge the atmosphere in the vicinity of the patch with nitrogen in the manufacturing method of [2] above, for example, it is possible to purge the atmosphere in the packaging bag with nitrogen by adopting a technique in which the step of conducting a heat sealing operation itself is conducted in a box or the like that is filled with nitrogen in a series of manufacturing steps.

In addition, if necessary, the oxygen absorbent may be loaded at the same time as or before or after loading of the patch, and it may be loaded at the stage after nitrogen purge in the case of further conducting nitrogen purge as well.

In addition, in the manufacturing method of [1] or [2] above, the step of discharging the gas (oxygen, nitrogen, and the like) in the bag-like article ([1]) or in the vicinity of the patch ([2]) to the outside to an extent that the two laminates tightly sandwich the patch by roll pass (an operation to allow the bag-like article ([1]) or the two laminates ([2]) sandwiching the patch to pass between two rolls), pressing, or suction degassing may be conducted as an arbitrary step before the opening is heat-sealed. Incidentally, it is preferable to conduct this step in a case in which a volatilizing substance is present in the bag-like article since it is preferable that a gas is not present in the bag-like article as much as possible.

<Patch>

Examples of the configuration of the patch to be a target of the present invention may include a patch equipped with at least a support, a layer containing a drug (rivastigmine or lidocaine), and a pressure sensitive adhesive layer or a patch equipped with at least a support and a pressure sensitive adhesive layer (drug-containing pressure sensitive adhesive layer) containing a drug (rivastigmine or lidocaine).

As described above, in the patch to be a target of the present invention, the drug-containing layer and the pressure sensitive adhesive layer may be separately fabricated or these layers may be fabricated as a single layer of a drug-containing pressure sensitive adhesive layer.

The patch preferably has the following configurations (i) to (iii).

(i) A patch including a support, a drug-containing layer which contains a drug and is provided on at least one surface of the support, and a pressure sensitive adhesive layer provided on the drug-containing layer.
(ii) A patch including a support, a pressure sensitive adhesive layer provided on at least one surface of the support, and a drug-containing layer which contains a drug and is provided on the pressure sensitive adhesive layer.
(iii) A patch including a support and a drug-containing pressure sensitive adhesive layer provided on at least one surface of the support.

[Support]

Examples of the support to be used in the patch to be a target of the present invention may include paper such as impregnated paper, coated paper, high-quality paper, kraft paper, Japanese paper, and glassine paper; plastic films such as a polyester film such as polyethylene terephthalate or polybutylene terephthalate, a polyolefin film such as polyethylene or polypropylene, a polyvinyl chloride film, a polycarbonate film, a polyurethane film, and a cellophane film; a foam body; fabrics such as a nonwoven fabric, a woven fabric, and a knitted fabric; and a laminate of two or more kinds thereof Examples of the material for the fabrics such as a nonwoven fabric, a woven fabric, and a knitted fabric may include polyester, polyurethane, polyethylene, and polypropylene.

In the patch to be a target of the present invention, for example, it is possible to preferably use a fabric selected from the group consisting of a nonwoven fabric, a woven fabric, and a knitted fabric. In addition, it is possible to preferably use a support formed of at least one kind of material selected from the group consisting of a polyester such as polyethylene terephthalate or polybutylene terephthalate and a polyolefin such as polyethylene or polypropylene.

Among them, a flexible material so as to be able to come in close contact with the skin and follow the movement of the skin and a material which can suppress the occurrence of skin rash after being attached for a long period of time are preferable. From the viewpoint of exhibiting excellent followability to movement of the skin surface while being attached, a knitted fabric and the like are preferable and a polyester knitted fabric is particularly preferable. In addition, the polyolefin film and polyester film described above are also suitably used.

In addition, it is desired that the drug (lidocaine or rivastigmine) contained in the drug-containing layer or the drug-containing pressure sensitive adhesive layer is not adsorbed to the support and the drug (lidocaine or rivastigmine) is not released from the support side. As described above, in order to suppress the adsorption and release of the drug, to improve the transdermal absorbability of the drug, and to suppress the occurrence of skin rash and the like, it is possible to adopt one that includes one or more layers of the materials described above and has a moisture permeability in a specific numerical range as the support. Specifically, the moisture permeability (to be measured at 40°C and 90% RH in conformity to JIS Z 0208) of the support is preferably 300 g/m²·24 hr or less and particularly preferably 50 g/m²·24 hr or less. By setting the moisture permeability of the support to be in the above range, the skin permeability of the drug (lidocaine or rivastigmine) increases.

In addition, it is preferable to adopt the form of a plastic film exhibiting excellent transparency from the viewpoint of preventing the patch from being noticeable at the time of being attached, that is, the color tone of the skin while being attached is easily seen through. By coloring the support such as a fabric to a color tone such as the skin color with a coloring agent, it is possible to decrease the difference between the support color and the skin color at the time of being attached. Examples of the coloring agent may include a pigment, an organic pigment, and a natural colourant.

The thickness of the support can be appropriately selected in consideration of physical properties such as elongation, tensile strength, workability, feel at the time of being attached, tightness of the diseased part, transfer of the drug to the support, and the like, but it is usually about from 5 μm to 1 mm.

In a case in which the support is a fabric, the thickness thereof is preferably from 50 μm to 1 mm, more preferably from 100 μm to 800 μm, and still more preferably from 200 μm to 700 μm, and the basis weight is preferably 300 g/m² or less, more preferably 200 g/m² or less, and still more preferably 150 g/m² or less from the viewpoint of followability to the skin.

In addition, in a case in which the support is a plastic film, the thickness thereof is preferably from 10 μm to

300 $\mu$m, more preferably from 12 $\mu$m to 200 $\mu$m, and still more preferably from 15 $\mu$m to 150 $\mu$m. However, when the thickness of the support is thinner than 5 $\mu$m, the strength and handling property of the patch deteriorate, thus the patch is hardly attached to the skin, is broken by contact with other members and the like, or peels off from the skin in a short time by contact with water at the time of bathing and the like in some cases. In addition, when the thickness of the support is too thick (more than 1 mm), it is difficult for the patch to follow the movement of the skin and it is easy to form a trigger to be peeled off at the peripheral portion of the patch, and it is thus concerned that the patch peels off from the skin in a short time or discomfort while being attached increases. In a case in which the support is a film, one surface or both surfaces of the support may be subjected to a treatment such as a sandblasting treatment or a corona treatment for the purpose of improving the anchoring property of the pressure sensitive adhesive with the support. In addition, unevenness may be provided on one surface or both surfaces of the support by a method other than sandblasting in order to make it easy to take out the patch from the packaging material (packaging bag or packaging body). A polyester film is preferable and a polyethylene terephthalate film is particularly preferable as a support which satisfies these conditions such as drug adsorptivity, moisture permeability, transparency, or thickness.

In addition, the support may contain additives such as an antistatic agent and an ultraviolet inhibitor to an extent that the effect of the present invention is not impaired. Examples of the antistatic agent may include surfactants (an anionic surfactant, a cationic surfactant, a nonionic surfactant, and an amphoteric surfactant). By antistatic agent, it is possible to eliminate the anchoring property of the support and process defects.

[Drug]

The drug to be applied to the patch to be a target of the present invention is not particularly limited. Examples of a systemic drug may include a corticosteroid, an analgesic anti-inflammatory agent, a hypnotic sedative, a tranquilizer, an antihypertensive agent, an antihypertensive diuretic, an antibiotic, a general anesthetic, an antibacterial agent, an antifungal agent, a vitamin agent, a coronary vasodilator, an antihistamine, an antitussive, a sex hormone, an antidepressant, a cerebral circulation ameliorating agent, an antiemetic agent, an antitumor agent, an enzyme agent, and a biopharmaceutical. In addition, examples of a local drug may include a dental antibiotic such as tetracycline hydrochloride, a disinfectant such as cetyl pyridinium chloride, an infective prophylactic and therapeutic agent such as chlorhexidine hydrochloride, an anti-inflammatory agent such as dimethylisopropylazulene, and an adrenocortical hormone such as hydrocortisone acetate.

Among them, particularly drugs susceptible to oxidation, volatilization, and adsorption are a target of the patch to be a target of the present invention. Incidentally, in the present invention, the "drug that volatilizes at room temperature" refers to a drug that volatilizes at from 1°C to 30°C. As the drug susceptible to oxidation, volatilization, and adsorption, for example, lidocaine of a local anesthetic and rivastigmine to be used as a therapeutic agent for Alzheimer type dementia (antidementia drug) are used as the target drug.

As lidocaine to be used in the present invention, lidocaine or a pharmaceutically acceptable salt thereof (lidocaine hydrochloride or the like) can be used. These may be contained singly or as a mixture, but it is preferable that lidocaine is singly contained in order to be contained in a drug-containing layer or a drug-containing pressure sensitive adhesive layer to be described later in a dissolved state.

In addition, rivastigmine ((S)-N-ethyl-3-[1-(dimethylamino)ethyl]-N-methyl-phenyl-carbamate) to be used in the present invention is in a free base form. A free base is a target drug of the present invention since it is more volatile and unstable than an acid addition salt. Rivastigmine has a free base form and an acid addition salt form. Rivastigmine in an acid addition salt form is also a target drug of the present invention in a case in which an acidic compound which converts an acid addition salt to a free base form is blended in a pressure sensitive adhesive or a drug-storing layer and a part or the whole of the acid addition salt is converted to a free base form.

[Drug-Containing Layer Containing Drug]

In the drug-containing layer which contains a drug, the drug is an essential constituent, and the drug-containing layer has a configuration in which the drug is contained in a matrix such as a polymer. The polymer is not particularly limited, but it is required to pay attention to the fact that a rubber-based or silicone-based polymer exhibits low drug retaining capacity depending on the kind of drug, thus the drug more easily volatilizes, and the stability of the drug is not maintained in some cases. Examples of the polymer to be used in the drug-containing layer may preferably include a (meth)acrylic acid alkyl ester resin.

Incidentally, in the patch to be a target of the present invention, the drug-containing layer is an arbitrary layer in a case in which there is a drug-containing pressure sensitive adhesive layer to be described later.

The content of the drug in the drug-containing layer is not particularly limited, but it is, for example, from 20 mass% to 95 mass%, preferably from 30 mass% to 90 mass%, and more preferably from 35 mass% to 90 mass% based on the total mass of the drug-containing layer.

In addition, the blended amount of the drug based on the total mass of the drug-containing layer and the pressure sensitive adhesive layer to be described later is, for example, from 10 mass% to 40 mass%, preferably from 10 mass% to 35 mass%, and more preferably from 10 mass% to 25 mass% in a case in which the drug is rivastigmine. In addition, the amount of the drug blended is from 0.1 mass% to 50 mass%, for example, from 1.0 mass% to 10 mass%, and particularly preferably from 2.0 mass% to 8.0 mass% in a case in which the drug is lidocaine.

The (meth)acrylic acid alkyl ester resin contained in the drug-containing layer plays a role as a thickening agent, and examples thereof may include a (meth)acrylic acid alkyl ester copolymer and an acrylic pressure sensitive adhesive containing a (meth)acrylic acid ester.

The configuration of the (meth)acrylic acid alkyl ester copolymer is not particularly limited, and the weight average molecular weight thereof is not particularly limited, but for example, a copolymer having a weight average molecular weight of from 10,000 to 300,000 and preferably from 100,000 to 200,000 can be used. A commercially available product can be suitably used as these, and for example, EUDRAGIT (registered trademark, manufactured by Evonik Rohm GmbH) products can be suitably used. Specific examples thereof may include EUDRAGIT E100, EPO, L100, L100-55, S100, RL100, RLPO, RS100, and RSPO and PLASTOID B.

Among these EUDRAGIT products, EUDRAGIT EPO is particularly preferable from the viewpoint of miscibility with the drug and adhesive property to the support.

As the acrylic pressure sensitive adhesive containing a (meth)acrylic acid ester, specifically, a polymer obtained by arbitrarily copolymerizing one kind or two or more kinds of copolymerizable monomers (for example, 2-ethylhexyl acrylate, vinyl pyrrolidone, vinyl acetate, methoxyethyl acrylate, hydroxyethyl acrylate, and acrylic acid) which contain one kind or two or more kinds of (meth)acrylic acid alkyl esters as the main component is preferable. (Meth)acrylic acid alkyl esters may be used singly or in combination of two or more kinds thereof. For example, n-butyl acrylate and methyl methacrylate can be used in combination. The weight average molecular weight of the (meth)acrylic acid ester to be used in the acrylic pressure sensitive adhesive containing a (meth)acrylic acid ester is not particularly limited, but it is from 100,000 to 1,000,000.

Incidentally, it is preferable that a component containing a hydroxyl group and also a carboxyl group is substantially not contained in the acrylic pressure sensitive adhesive containing a (meth)acrylic acid ester. However, a component containing a hydroxyl group and also a carboxyl group may be contained as long as the amount of the drug adsorbed to the packaging bag is less than 1% in the test item of <Content of Drug Adsorbed to Packaging Bag> to be described later. In the case of containing a component containing a hydroxyl group and also a carboxyl group, the contained form thereof may be a copolymerized state with a hydroxyl group-containing acrylic pressure sensitive adhesive or a blended state with a hydroxyl group-containing acrylic pressure sensitive adhesive. Incidentally, the hydroxyl group is not included in the carboxyl group, that is, the carbonyl group is not bonded to the carbon atom to which the hydroxyl group is bonded.

The content of the (meth)acrylic acid alkyl ester resin in the drug-containing layer is not particularly limited, but it is, for example, from 5 mass% to 25 mass%, preferably from 5 mass% to 20 mass%, and more preferably from 5 mass% to 15 mass% based on the total mass of the drug-containing layer in the case of a (meth)acrylic acid alkyl ester copolymer. The content is, for example, from 20 mass% to 90 mass%, preferably from 30 mass% to 85 mass%, and more preferably from 40 mass% to 80 mass% based on the total mass of the drug-containing layer in the case of an acrylic pressure sensitive adhesive containing a (meth)acrylic acid ester.

In addition, the blended amount of the (meth)acrylic acid alkyl ester resin based on the total mass of the drug-containing layer and the pressure sensitive adhesive layer to be described later is, for example, from 0.5 mass% to 5.0 mass%, preferably from 1.0 mass% to 4.0 mass%, and more preferably from 1.0 mass% to 3.0 mass% in the case of a (meth)acrylic acid alkyl ester copolymer. The blended amount is, for example, from 40 mass% to 90 mass%, preferably from 50 mass% to 90 mass%, and more preferably from 60 mass% to 90 mass% in the case of an acrylic pressure sensitive adhesive containing a (meth)acrylic acid ester.

In the present invention, the drug-containing layer may further contain, other additives such as a softening agent (plasticizer) and an inorganic filler if desired.

In the case of containing other additives, the blended amount thereof is, for example, from 0 mass% to 40 mass% or from 0 mass% to 30 mass% and preferably from 0 mass% to 20 mass% based on the total mass of the drug-containing layer. In addition, the blended amount of the other additives based on the total mass of the drug-containing layer and the pressure sensitive adhesive layer to be described later is, for example, from 0 mass% to 30 mass% or from 0 mass% to 20 mass% and preferably from 0 mass% to 10 mass%.

[Pressure Sensitive Adhesive Layer]

In the patch to be a target of the present invention, the pressure sensitive adhesive constituting the pressure sensitive adhesive layer is not particularly limited, but a rubber-based or silicone-based polymer exhibits low drug retaining capacity depending on the kind of drug, thus the drug more easily volatilizes, and the stability of the drug is not maintained in some cases. Accordingly, the pressure sensitive adhesive layer particularly preferably contains a (meth)acrylic acid alkyl ester-based pressure sensitive adhesive depending on the kind of drug. Particularly in consideration of stability of the drug, the pressure sensitive adhesive layer is preferably composed of an acrylic pressure sensitive adhesive containing a (meth)acrylic acid ester having a hydroxyl group.

As the acrylic pressure sensitive adhesive, specifically, a polymer which contains one kind or two or more kinds of (meth) acrylic acid hydroxyalkyl esters and is obtained by arbitrarily copolymerizing one kind or two or more kinds of copolymerizable monomers (for example, 2-ethylhexyl acrylate, vinyl pyrrolidone, vinyl acetate, methoxyethyl acrylate, hydroxyethyl acrylate, and acrylic acid) is preferable.

As the acrylic pressure sensitive adhesive containing an acrylic acid ester having a hydroxyl group, it is possible to suitably use DURO-TAK (registered trademark) 87-202A, 87-208A, 87-2510, 87-208A, 87-2287, 87-4287, 87-2516, and 87-2525 (Henkel) and the like of a commercially available product.

Incidentally, as the acrylic pressure sensitive adhesive, it is preferable not to substantially contain an acrylic pressure sensitive adhesive containing a carboxyl group, and the acrylic pressure sensitive adhesive containing a carboxyl group is desirably 5 mass% or less with respect to the total mass of the pressure sensitive adhesive layer even in the case of containing it.

In the present invention, the mass proportion of the pressure sensitive adhesive layer to the total mass of the drug-containing layer and the pressure sensitive adhesive layer is, for example, from 40 mass% to 95 mass%, preferably from 50 mass% to 90 mass%, and more preferably from 60 mass% to 90 mass%.

In the present invention, the pressure sensitive adhesive layer may further contain other drugs and other additives such as a tackifier, a crosslinking agent, a softening agent (plasticizer), an absorption promoter, a polyhydric alcohol, silicone oil, an inorganic filler, and an ultraviolet absorber if desired.

Examples of the tackifier may include a terpene-based tackifier, a terpene phenol-based tackifier, a cumarone indene-based tackifier, a styrene-based tackifier, a rosin-based tackifier, a xylene-based tackifier, a phenol-based tackifier, and a petroleum-based tackifier.

Various crosslinking agents can be further added to the pressure sensitive adhesive layer for the purpose of enhancing the cohesive force of the acrylic pressure sensitive adhesive. Examples of the crosslinking agent may include a polyfunctional isocyanate compound, a polyfunctional epoxy compound, and a polyvalent metal salt. Specifically, polyisocyanate [for example, Coronate (registered trademark) HL (hexamethylene diisocyanate HDI-TMP adduct, manufactured by Nippon Polyurethane Industry Co., Ltd.)] is preferable. In addition, it is possible to blend calcium carbonate, magnesium carbonate, a silicate salt, zinc oxide, titanium oxide, magnesium sulfate, calcium sulfate, and the like as the filler.

Examples of the absorption promoter may include terpene oil such as d-limonene, fatty acid esters such as glycerin monolaurate, glycerin monooleate, and diethyl sebacate, and fatty acids such as azone, prothiodecane, oleic acid, lauric acid, and myristic acid or derivatives thereof.

The blending of these additives is arbitrary, but the blended amount of other additives based on the total mass of the pressure sensitive adhesive layer and the drug-containing layer is, for example, from 0 mass% to 40 mass% and preferably from 0 mass% to 30 mass%.

The antioxidant is not necessarily required in the patch to be a target of the present invention. There are cases in which antioxidants are added to commercially available pressure sensitive adhesives in advance by suppliers, but it is defined that an antioxidant is not added since the amount thereof is usually significantly small. Examples of the antioxidant may include dibutylhydroxytoluene, tocopherol acetate, ascorbic acid, benzoic acid, a paraben, benzalkonium chloride, and benzethonium chloride.

[Drug-Containing Pressure Sensitive Adhesive Layer]

In the present invention, the pressure sensitive adhesive layer may contain a drug, that is, it may be a drug-containing pressure sensitive adhesive layer. In that case, the drug-containing layer described above is an arbitrary layer.

In the case of the drug-containing pressure sensitive adhesive layer as well, the pressure sensitive adhesive described in the "Pressure Sensitive Adhesive Layer" can be suitably used. However, depending on the kind of drug, it is required to adopt a pressure sensitive adhesive that can be coated at a low temperature or to consider selecting a pressure sensitive adhesive that can be coated without a solvent so that the drug does not volatilize at the time of coating the pressure sensitive adhesive.

The blended amount of the drug based on the total mass of the drug-containing pressure sensitive adhesive layer is, for example, from 10 mass% to 40 mass%, preferably from 10 mass% to 35 mass%, and more preferably from 10 mass% to 25 mass% in the case of rivastigmine. In addition, the blended amount of the drug is from 0.1 mass% to 50 mass%, for example, from 1.5 mass% to 4.5 mass%, particularly preferably from 2.0 mass% to 3.5 mass% in the case of lidocaine.

In addition, in a case in which there is a drug-containing layer in addition to the drug-containing pressure sensitive adhesive layer, the mass proportion of the drug-containing pressure sensitive adhesive layer to the total mass of the drug-containing pressure sensitive adhesive layer and the drug-containing layer is, for example, from 40 mass% to 95 mass%, preferably from 50 mass% to 90 mass%, and more preferably from 60 mass% to 90 mass%.

In addition, the drug-containing pressure sensitive adhesive layer may further contain the other additives described in the "Pressure Sensitive Adhesive Layer". The amount of other additives blended in the drug-containing pressure sensitive adhesive layer is arbitrary, but it is, for example, from 0 mass% to 40 mass% and preferably from 0 mass% to 30 mass% based on the total mass of the drug-containing pressure sensitive adhesive layer.

[Rubber-Based Pressure Sensitive Adhesive Layer]

As described above, in the patch to be a target of the present invention, an acrylic pressure sensitive adhesive is preferable as the pressure sensitive adhesive constituting the pressure sensitive adhesive layer depending on the kind of drug (for example, rivastigmine). However, a rubber-based pressure sensitive adhesive can also be used. A rubber-based pressure sensitive adhesive may be adopted, for example, in a case in which the drug is lidocaine.

(Rubber-Based Elastomer)

As the rubber-based elastomer to be contained in the rubber-based pressure sensitive adhesive, it is possible to apply various thermoplastic elastomers such as a thermoplastic block copolymer such as a styrene-isoprene-styrene copolymer (hereinafter, referred to as "SIS" in some cases), a styrene-butadiene-styrene copolymer (hereinafter, referred to as "SBS" in some cases), or a hydrogenated product thereof, a styrene-ethylene-propylene-styrene copolymer (hereinafter, referred to as "SEPS" in some cases), or a styrene-ethylene-butylene-styrene copolymer (hereinafter, referred to as "SEBS" in some cases); an ethylene-vinyl acetate copolymer; and an ethylene-$\alpha$-olefin copolymer. Among them, a styrene-based thermoplastic elastomer that is a thermoplastic block copolymer such as SIS, SBS, SEPS, or SEBS is suitably used since it exhibits excellent tackiness and cohesive property.

In particular, SIS can play a role to retain each component in the pressure sensitive adhesive layer and to dissolve or disperse other components as well as to impart elasticity to the pressure sensitive adhesive layer (plaster layer), and it is thus particularly suitably applied to the pressure sensitive adhesive layer of the patch to be a target of the present invention. In the patch to be a target of the present invention, the content of SIS is not particularly limited, but it is preferably from 15 mass% to 50 mass% and more preferably from 20 mass% to 40 mass% when the total mass of the pressure sensitive adhesive layer is taken as 100 mass%.

Incidentally, SIS to be used in the present invention is not particularly limited, but usually the mass ratio (styrene/isoprene) of styrene to isoprene is from 10/90 to 30/70 when the total mass of styrene and isoprene is taken as 100 parts by mass, but it is preferably from 20/80 to 25/75. It is easy to fabricate a rubber-based pressure sensitive adhesive by using a SIS block copolymer having a high isoprene mass ratio as described above. As SIS, a commercially available styrene-isoprene-styrene block copolymer may be used, and examples thereof may include JSR SIS 5002 (JSR Corporation).

(Highly Polar Resin and Low-Polarity Resin)

It is preferable to blend a highly polar resin in the rubber-based pressure sensitive adhesive layer in order to control the skin permeability of the drug, and a low-polarity resin may be further blended therein in order to adjust the skin permeability. Incidentally, it is desirable to select these highly polar resin and low-polarity resin are from conventionally known tackifying resins to be used in order to enhance the viscosity of the pressure sensitive adhesive layer.

The highly polar resin refers to a resin having a polar group such as a carboxyl group, an amino group, an ester group, or an amide group. It is preferable to use a tackifying resin containing an ester group in consideration of low irritation of the patch to be a target of the present invention. Examples of such a highly polar resin may include a rosin resin, a rosin ester resin, and resins of other rosin derivatives, and those having a polar group among terpene phenolic resins. Among them, hydrogenated rosin glycerin ester is preferably used, and examples of the commercially available product thereof may include PINECRYSTAL KE-311 (manufactured by Arakawa Chemical Industries, Ltd.).

The low-polarity resin refers to a resin which does not have a polar group, and examples of such a low-polarity resin may include tackifying resins which does not have a polar group among a terpene resin, a coumarone indene resin, a petroleum resin, and a dicyclopentadiene resin. Among them, a terpene resin is preferably used, and examples of the commercially available product thereof may include YS Resin (manufactured by YASUHARA CHEMICAL CO., LTD.).

By blending at least a highly polar resin in the rubber-based pressure sensitive adhesive layer, the skin permeability of the drug to be blended is improved. By further blending a low-polarity resin, the skin permeability of the drug is controlled and the medicinal ingredient can be sustained. In other words, by blending a highly polar resin and a low-polarity resin in the pressure sensitive adhesive layer in appropriate amounts, it is possible to continuously administer the medicinal ingredient for a desired period of time, to decrease the residual amount of the medicinal ingredient in the pressure sensitive adhesive layer after being attached, and to realize efficient drug administration. Here, the blended proportion (mass ratio) of the highly polar resin/the low-polarity resin can be set to be in a range of from 30.0/70.0 to 100.0/0 (mass ratio) when the total mass of the highly polar resin and the low-polarity resin is taken as 100 parts by mass. There is a tendency that the skin permeability is not stably sustained in a case in which the low-polarity resin is blended in a great amount and the highly polar resin is blended in a small amount. A preferable blended ratio (mass ratio) is from 40.0/60.0 to 100.0/0, and a more preferable blended ratio (mass ratio) is from 51.0/49.0 to 100.0/0. In addition, the total mass content of both is in a range of from 10 to 50 mass%, preferably from 15 to 40 mass%, and more preferably from 20 to 35 mass% when the total mass of the pressure sensitive adhesive layer is taken as 100 mass%.

(Softening Agent)

In the rubber-based pressure sensitive adhesive layer, a softening agent may be used in order to improve the handling property of the pressure sensitive adhesive. Examples of the softening agent (plasticizer) may include a petroleum-based softening agent such as liquid paraffin; a liquid rubber-based softening agent such as liquid polyisoprene, polybutene, or polyisobutylene; a dibasic acid ester-based plasticizer such as a phthalic acid ester or an adipic acid ester; and other plasticizers such as polyethylene glycol and a citric acid ester. Among them, liquid paraffin may be preferably used since it exhibits excellent compatibility with the rubber-based elastomer and it is not concerned that liquid paraffin decreases the cohesive force of the rubber-based elastomer. Examples of a commercially available product thereof may include HICALL (registered trademark, liquid paraffin manufactured by Kaneda Corporation) M series. From the viewpoint of tackiness, the content of these softening agents is preferably in a range of from 25 mass% to 47 mass% and more preferably from 30 mass% to 42 mass% when the total mass of the pressure sensitive adhesive layer is taken as 100 mass%.

(Other Additives)

The rubber-based pressure sensitive adhesive layer may further contain additives such as an oxidation inhibitor (antioxidant), a filler, a pigment, a drug stabilizer, a drug solubility improver, and a drug solubility inhibitor usually except a transdermal absorption promoter to be blended in the pressure sensitive adhesive layer of the transdermal absorption type preparation if necessary. These additives may be used singly or in combination of two or more kinds thereof. It is required to consider so that the residual rate does not increase high in addition to skin permeability. For example, it is preferable not to add a surfactant from the viewpoint of skin irritation and residual rate.
As commonly used transdermal absorption promoter, for example, there are an aliphatic alcohol, a fatty acid, a fatty acid ester, an alcohol amine, a polyhydric alcohol alkyl ether, a polyoxyethylene alkyl ether, glyceride (namely, fatty acid ester of glycerin), a polyhydric alcohol medium chain fatty acid ester, a lactic acid alkyl ester, a dibasic acid alkyl ester, an acylated amino acid, and a pyrrolidone. Usually, these transdermal absorption promoters are used singly or in combination of two or more kinds thereof.
An example of the configuration of a composition of the rubber-based pressure sensitive adhesive layer is that the rubber-based elastomer is from 15 mass% to 50 mass%, the total mass of the highly polar resin and the low-polarity resin is from 10 mass% to 50 mass%, the softening agent is from 25 mass% to 47 mass%, and the drug (particularly in the case of lidocaine) is from 1.5 mass% to 4.5 mass% when the total mass of the pressure sensitive adhesive layer is taken as 100 mass%. Preferably, the rubber-based elastomer is from 20 mass% to 40 mass%, the total mass of the highly polar resin and the low-polarity resin is from 15 mass% to 40 mass%, the softening agent is from 30 mass% to 42 mass%, and the drug (particularly in the case of lidocaine) is from 2.0 mass% to 3.5 mass% when the total mass of the pressure sensitive adhesive layer is taken as 100 mass%.

[Thickness of Each Layer]

In the case of an aspect that the drug-containing layer and the pressure sensitive adhesive layer are separately configured in the patch, the thickness of the drug-containing layer can be usually set to from 10 μm to 80 μm and the thickness of the pressure sensitive adhesive layer can be usually set to from 10 μm to 100 μm.
In addition, in the case of an aspect that the drug-containing layer and the pressure sensitive adhesive layer are configured as one layer of the drug-containing pressure sensitive adhesive layer, the thickness of the pressure sensitive adhesive layer is usually in a range of from 20 μm to 500 μm, preferably from 30 μm to 400 μm, more preferably from 35 μm to 300 μm, and still more preferably from 40 μm to 200 μm.
When the thicknesses of the pressure sensitive adhesive layer and drug-containing pressure sensitive adhesive layer are too thin, the skin tackiness is insufficient or the content (absolute amount) of the drug is low, and the persistence of skin absorption of the drug is thus insufficient. On the other hand, when the thicknesses of the pressure sensitive adhesive layer and drug-containing pressure sensitive adhesive layer are too thick, a large amount of pressure sensitive adhesive may remain on the skin at the time of peeling off and handling property deteriorates in some cases. In addition, it is concerned that the solvent remains and the skin irritation increases in the case of coating the pressure sensitive adhesive layer by a dissolution coating method in the manufacture of the patch as to be described later.

In addition, when the thickness of the drug-containing layer is too thin, the drug content (absolute amount) is low, and the persistence of skin absorption of the drug is thus insufficient.

[Separator Layer]

The patch to be a target of the present invention includes at least the support and the drug-containing pressure sensitive adhesive layer or the support, the drug-containing layer, and the pressure sensitive adhesive layer as described above, but a separator layer may be provided to the patch if necessary. Usually, the patch often has a layer configuration of support/pressure sensitive adhesive layer (drug-containing pressure sensitive adhesive layer, pressure sensitive adhesive layer, and the like)/separator layer.

The "separator layer" means a layer (also referred to as a release layer, a release liner, or the like) that protects the layer (pressure sensitive adhesive layer, drug-containing layer, or drug-containing pressure sensitive adhesive layer) to be in contact with the skin until the patch is attached to the skin, prevents the degeneration, and is peeled off when attaching the patch to the skin, and it is commonly used in patches. The separator layer is preferably formed of a material to which the drug or the like in the drug-containing layer or the drug-containing pressure sensitive adhesive layer is hardly absorbed or adsorbed. The material can be appropriately selected in further consideration of releasability from these pressure sensitive adhesive layer and the like, flexibility, and the like. It is possible to use, for example, a plastic film such as polyethylene terephthalate (PET), polybutylene terephthalate, polyethylene naphthalate, non-oriented polypropylene, oriented polypropylene, polyethylene, polyurethane, polyvinyl chloride, or polystyrene or a colorless or colored sheet such as a synthetic resin, synthetic paper, silicone-processed paper obtained by subjecting a synthetic fiber to silicone processing, an aluminum foil, or laminated paper obtained by laminating polyethylene or the like on kraft paper as the separator layer. In particular, a release-treated resin film such as a silicone-treated PET film, a silicone-treated polypropylene film, a silicone-treated polyethylene film, a fluororesin-coated PET film, a fluororesin-coated polypropylene film, or a fluororesin-coated polyethylene film is preferably used as the separator layer. A laminate of these release-treated resin films with paper, a resin film vapor-deposited with aluminum, paper coated with silicone oil or the like, and the like may be used. Incidentally, it is possible to subject not only the surface in contact with the layer (pressure sensitive adhesive layer, drug-containing layer, or drug-containing pressure sensitive adhesive layer) to be in contact with the skin of the patch but also the surface on the side opposite to these layers to the release treatment of the separator layer. In addition, uneven shapes may be provided on one surface or both surfaces of the separator layer in order to make it easy to take out the patch from the packaging material (packaging bag or packaging body). In addition, the shape of the separator layer can be rectangular, quadrate, circular, and the like, and it can have a rounded corner if desired. The size of the separator layer is the same as or slightly larger than the size of the support in the patch (lidocaine-containing patch or rivastigmine-containing patch). The separator layer may be composed of one sheet or a plurality of sheets divided. The notch thereof may be composed of a straight line, a wavy line, or a perforated line shape, or it may be in a state in which parts of the separator layers overlap each other. The thickness of the separator layer is not particularly limited, but it is usually in a range of from 10 $\mu$m to 1 mm, preferably from 20 $\mu$m to 500 $\mu$m, and more preferably from 40 $\mu$m to 200 $\mu$m.

<Method of Manufacturing Patch>

The method of manufacturing the patch to be a target of the present invention is not particularly limited. For example, it is possible to adopt a hot melt method that is a general method of manufacturing a patch, a calendaring method, a dissolution method, or the like. Incidentally, it is preferable not to intentionally add moisture to the pressure sensitive adhesive layer (plaster) in the manufacturing process particularly in the case of a lidocaine-containing patch.

Among them, in the case of an aspect that the drug-containing layer and the pressure sensitive adhesive layer are separately configured, the patch to be a target of the present invention can be suitably manufactured by the following process i) or ii).

i) A drug-containing layer forming step of forming a layer containing a drug by coating a solution containing the drug on a support, a pressure sensitive adhesive layer forming step of forming a pressure sensitive adhesive layer by coating a solution containing a pressure sensitive adhesive (for example, an acrylic pressure sensitive adhesive) on a separator layer, and a patch forming step of pasting the drug-containing layer formed on the support and the pressure sensitive adhesive layer formed on the separator layer to each other.

ii) A pressure sensitive adhesive layer forming step of forming a pressure sensitive adhesive layer by coating a solution containing a pressure sensitive adhesive (for example, an acrylic pressure sensitive adhesive) on a support, a drug-containing layer forming step of forming a layer containing a drug by coating a solution containing the drug on a separator layer, and a patch forming step of pasting the drug-containing layer formed on the separator layer and the pressure sensitive adhesive layer formed on the support to each other.

Alternatively, in the case of an aspect that the drug-containing layer and the pressure sensitive adhesive layer are configured as one layer of the drug-containing pressure sensitive adhesive layer, the patch to be a target of the present invention can be suitably manufactured by the following process iii).

iii) A pressure sensitive adhesive layer forming step of forming a drug-containing pressure sensitive adhesive layer by coating a solution containing a pressure sensitive adhesive and a drug on a separator layer and a patch forming step of pasting the pressure sensitive adhesive layer and a support to each other.

Incidentally, in the case of selecting rivastigmine as the drug of the patch, from the viewpoint of stability of the drug, it is preferable to form the a drug-containing layer by a so-called "ointment coating method" in which the operation is conducted by avoiding heating as much as possible in the mixing and drying steps instead of a calendering method or hot melt method which includes a heating and kneading (stirring) step and is adopted for the fabrication of a usual patch or a dissolution coating method including a heating and drying step after coating. The "ointment coating method" refers to a method of forming a layer at a low temperature, for example, under a temperature condition of 1°C or higher and lower than 60°C, for example, at room temperature. Incidentally, it is preferable to select those having a low weight average molecular weight (for example, a (meth)acrylic acid alkyl ester copolymer having a weight average molecular weight of 300,000 or less) as a resin (for example, (meth)acrylic acid alkyl ester resin) in the drug-containing layer containing rivastigmine described above since it can be coated at a lower temperature.

In addition, the step of forming the pressure sensitive adhesive layer can be conducted by any of the conventional methods of forming a pressure sensitive adhesive layer, for example, the dissolution coating method described above, an emulsion method, a hot melt method, and an electron beam curing method.

In addition, it is preferable to adopt a hot melt method in a case in which the pressure sensitive adhesive layer is composed of a rubber-based pressure sensitive adhesive. In the hot melt method, a rubber-based elastomer, a highly polar resin, a low-polarity resin, a softening agent, and other additives are first heated and stirred at a high speed and a pressure sensitive adhesive (plaster) temperature of from 100°C to 200°C for from 20 minutes to 100 minutes in a nitrogen atmosphere by using a high-speed rotary mixer capable of controlling heating to obtain a molten material. Thereafter, a drug is added to the molten material, and the mixture is further heated and stirred at a high speed and a plaster temperature of from 100°C to 150°C for from 5 minutes to 30 minutes to obtain a pressure sensitive adhesive in which the respective components are uniformly mixed. Incidentally, the temperature and the time at the time of stirring described above are an example, and they are not limited to these ranges.

The pressure sensitive adhesive (plaster) obtained by the above method is extruded through a die head portion of which the temperature is controlled at from 100°C to 150°C by using a hot melt coating machine and spread on the separator layer so as to have a coating amount of from 100 g/$m^2$ to 250 g/$m^2$. Thereafter, a support is laminated on this, and the resultant laminate is cut into a predetermined shape, whereby a patch is fabricated.

The shape of the patch is not particularly limited, and various shapes such as a rectangle (regular square, oblong, or the like), a quadrangle (trapezoid, rhombus, or the like), a polygon, a circle, an ellipse, a semicircle, a triangle, a crescent, and a shape formed by combining these can be selected according to the place to be attached.

Incidentally, the area of the patch can be appropriately determined, but it is from 40 $cm^2$ to 240 $cm^2$, for example, in the case of a lidocaine-containing patch and it is from 2 $cm^2$ to 25 $cm^2$, for example, in the case of a rivastigmine-containing patch in consideration of the administration purpose and dosage of the drug, a decrease in amount of the drug which volatilizes in the packaging material and adheres to the support as much as possible, and easy attachment.

Incidentally, it is preferable that the patch to be a target of the present invention fabricated as described above is housed in the packaging body fabricated by using the packaging material of the present invention and stored until immediately before being used.

[Administration Method: Lidocaine-Containing Patch]

A preferred dosage of the active ingredient (lidocaine) to be administered by the lidocaine-containing patch to be a target of the present invention is generally that the blended amount of the drug based on the total mass of the drug-containing layer and the pressure sensitive adhesive layer is from 0.1 mass% to 50 mass%, for example, from 1.0 mass% to 10 mass%, and particularly preferably from 2.0 mass% to 8.0 mass%. With regard to the number of dose, the lidocaine-containing patch can be administered from 1 time to 2 times per day. The administration time can be set to, for example, a maximum of 12 hours. The lidocaine-containing patch can be applied to the skin for an arbitrary time from morning to night if necessary. For example, it can be attached at 7 o'clock in the morning and peeled off at 7 o'clock in the evening or it can be attached at 8 o'clock in the evening after bathing and peeled off at 8 o'clock in the next morning.

[Administration Method: Rivastigmine-Containing Patch]

The amount of the active ingredient (rivastigmine) to be administered in the treatment of cerebral and neuronal disorders by the rivastigmine-containing patch to be a target of the invention depends on the nature and severity of the complaint to be treated and the weight of the patient. The preferred unit dosage generally contains from 0.25 mg to 700 mg, advantageously from 0.5 mg to 300 mg, preferably from 1 mg to 150 mg, and for example, between 2 mg and 50 mg, namely 2 mg, 5 mg, 10 mg, 15 mg, 20 mg, 25 mg, 30 mg, 40 mg, or 50 mg of the product (rivastigmine). This unit dosage is administered usually one or more times per day, for example, 2 times, 3 times, 4 times, or 5 times per day, and preferably from 1 time to 3 times per day. The total dosage for one person varies between 0.5 mg and 1400 mg per day, advantageously between 1 mg and 900 mg per day, for example, between 2 mg and 500 mg, and more conveniently between 4 mg and 10 mg per day.

Examples

Hereinafter, the present invention will be specifically described with reference to Examples and Comparative Examples, but the present invention is not limited to these Examples.

<Lidocaine-Containing Preparation>

The drug (lidocaine) stability of the patch product of the present invention was evaluated based on the lidocaine content in the pressure sensitive adhesive component of the patch and the amount of lidocaine adsorbed to the packaging bag.

[Drug Content]

The patch products fabricated in Example 1 and Comparative Example 1 to be described later were stored for 1 month under a severe condition (60°C), and the drug content (residual amount of lidocaine) in the pressure sensitive adhesive component of the patch immediately after being manufactured and after being stored for predetermined periods of time (after 1 week, after 2 weeks, and after 1 month) was measured in conformity to the following [Content Measurement Procedure (1)]. The results thus obtained are presented in Table 1.

[Content Measurement Procedure (1)]

<Amount of Drug Remaining in Patch (1)>

Immediately after manufacture and after the lapse of predetermined periods of time, the patch was taken out from the packaging bag of the patch product, the release paper was peeled off from the patch, the patch was then immersed in a sealable glass container containing an internal standard solution and tetrahydrofuran for HPLC to dissolve the pressure sensitive adhesive, and a mixed solution of acetonitrile for pharmaceutical/sodium dihydrogen phosphate buffer (pH: 3) was then added to the container to prepare a sample solution (patch).
Separately, a standard solution was prepared by using standard lidocaine through the same operation.
These sample solution and standard solution were analyzed together by a high performance liquid chromatography (HPLC) method. The amount of lidocaine (drug content in patch) in the sample (patch) was calculated from the ratio of the peak area of lidocaine to the peak area of the internal standard substance in the standard solution and the sample solution (patch).

<Evaluation>

From the results thus obtained, the residual amount of drug (%) was calculated based on the following formula, and the residual amount of drug in the patch was calculated from this value as an average value of 3 times of measurement.

$$\text{Residual amount of drug (\%)} = [\text{amount of lidocaine after lapse of}$$

$$\text{predetermined period of time/amount of lidocaine at the time of manufacture (initial}$$

$$\text{value})] \times 100$$

[Adsorbed Amount of Drug]

The amount of lidocaine adsorbed to the packaging bag when the patch products fabricated in Example 1 and Comparative Example 2 to be described later were stored under a severe condition (60°C) for 7 days and 14 days was measured and evaluated in conformity to the following [Content Measurement Procedure (2)]. The results thus obtained are presented in Table 2.

[Content Measurement Procedure (2)]

<Amount of Drug Remaining in Patch (2)>

In conformity to the procedure of <Amount of Drug Remaining in Patch (1)> described above, the amount of lidocaine (drug content in patch) in the sample (patch) after being stored for predetermined periods of time (after 7 days and after 14 days) was calculated.

<Amount of Drug Adsorbed to Packaging Bag>

The packaging bag of the patch product stored under a severe condition was finely cut and used to prepare a sample solution (packaging bag) in conformity to the procedure of <Amount of Drug Remaining in Patch (1)> described above, and the amount of lidocaine (drug content in packaging bag) in the sample (packaging bag) was calculated by the same procedure using a standard solution.

<Evaluation>

From the results thus obtained, the adsorbed amount of drug (%) was calculated based on the following formula, and the average value of 3 times of measurement was calculated from this value and evaluated as follows.

$$\text{Adsorbed amount of drug (\%)} = [\text{drug content in packaging bag/(drug content}$$

$$\text{in packaging bag + drug content in patch)}] \times 100$$

Judgment ○: amount of drug (lidocaine) adsorbed to packaging bag is less than 1%
Judgment ✕: amount of drug (lidocaine) adsorbed to packaging bag is 1% or more

[Example 1]

By the hot melt method described in the [Method of Manufacturing Patch], lidocaine, SIS 5002 [styrene content: 22 mass%, diblock rate: 15 mass%, manufactured by JSR Corporation] as SIS, PINECRYSTAL KE-311 [hydrogenated rosin glycerin ester, manufactured by Arakawa Chemical Industries, Ltd.] as a highly polar resin, YS Resin PX1150N [terpene resin, manufactured by YASUHARA CHEMICAL CO., LTD.] as a low-polarity resin, and HICALL (registered trademark) M-352 [liquid paraffin, manufactured by Kaneda Corporation] as a softening agent were heated and stirred at the respective blending proportions (the numerical value (mass%) was a numerical value when the total mass of the pressure sensitive adhesive layer was taken as 100 mass%, the same applies hereinafter) of 3.0 mass%, 32.0 mass%, 17.5 mass%, 10.0 mass%, and 37.5 mass% to prepare a uniform pressure sensitive adhesive composition. Incidentally, the heating and stirring were conducted as follows. The above-described materials other than the drug were mixed in a Henschel mixer in a nitrogen atmosphere at a temperature of 170°C and the number of revolutions of 600 rpm, after the mixture reached a uniform state, the temperature thereof was set at 140°C, and the mixture was stirred for 15 minutes at the number of revolutions of 200 rpm.
Subsequently, a pressure sensitive adhesive layer was formed by spreading the pressure sensitive adhesive composition on a silicone-treated polyester film (thickness: 75 $\mu$m) to have a thickness of 200 g/m$^2$. A polyester knitted

fabric (circular knit, weight per unit area: about 100 g/m$^2$, thickness: about 500 $\mu$m) as a support was laminated on this pressure sensitive adhesive layer, and the resultant laminate was cut, thereby fabricating a patch.

Incidentally, the mass ratio of highly polar resin/low-polarity resin (a numerical value when the total mass of the highly polar resin and the low-polarity resin was taken as 100 mass%) of Example 1 was 82.5/17.5.

The patch thus fabricated was enclosed in a packaging bag obtained by allowing laminates (see below) having a layer configuration in which a COC film on the innermost layer side, an aluminum film, and a polyester film were laminated in this order to face each other and heat-sealing three sides of the facing laminates, and the rest one side of the packaging bag was heat-sealed, thereby fabricating a patch product (product size: 175 mm x 135 mm, four sides were heat-sealed, sealed width: 7 mm).

Incidentally, the details of the laminate used in Example 1 are as follows.

Laminate configuration: COC film/adhesive/aluminum film (AL)/adhesive/polyester film (PET)
COC film: cyclic olefin copolymer, thickness: 30 $\mu$m
Polyester film: polyethylene terephthalate film, thickness: 12 $\mu$m
Aluminum film: aluminum foil, thickness: 7 $\mu$m
Adhesive: polyurethane for adhesive

[Comparative Example 1]

A patch product was fabricated by the same method as in Example 1 except that the innermost layer inside the packaging bag was changed to EVOH (thickness: 30 $\mu$m).

[Comparative Example 2]

A patch product was fabricated by the same method as in Example 1 except that the innermost layer inside the packaging bag was changed to a polyethylene film (thickness: 30 $\mu$m).

[Table 1]

Table 1

| | | Initial value | After 1 week | After 2 weeks | After 1 month |
|---|---|---|---|---|---|
| Residual amount of drug in patch (%) [stored at 60°C] | Example 1 | 100 | 100 | 100 | 99 |
| | Comparative Example 1 | 100 | 97 | 96 | 96 |

[Table 2]

Table 2

| | | After 7 days | After 14 days |
|---|---|---|---|
| Amount of drug adsorbed to packaging bag [stored at 60°C] | Example 1 | ○ | ○ |
| | Comparative Example 2 | × | × |

From the results in Table 1, it can be seen that a result that the temporal stability of lidocaine is significantly excellent even under a severe condition of 60°C has been obtained in Example 1 in which a lidocaine-containing patch is enclosed in a packaging bag composed of a laminate having a COC film layer as the innermost layer.

On the other hand, a poor result that the patch is lacking in temporal stability as a decrease in content of lidocaine in the plaster is revealed has been obtained in Comparative Example 1 of a case using an EVOH film.

From the results in Table 2, it can be seen that a result that the temporal stability of lidocaine is significantly excellent as the adsorption of drug to the packaging bag is minor (the adsorbed amount of drug is less than 1%) even after the lapse of 14 days under a severe condition of 60°C has been obtained in Example 1 in which a lidocaine-containing patch is enclosed in a packaging bag composed of a laminate having a COC film layer as the innermost layer.

On the other hand, a poor result that the patch is lacking in temporal stability as the amount of drug adsorbed to the packaging bag already exceeds 1% after the lapse of 7 days and a large amount of the drug is adsorbed to the packaging bag has been obtained in Comparative Example 2 of a case using a polyethylene film.

<Rivastigmine-Containing Preparation>

The drug (rivastigmine) stability of the patch product of the present invention was evaluated based on the amount of rivastigmine adsorbed to the packaging bag after the lapse of a predetermined period of time.

[Adsorbed Amount of Drug]

The amount of rivastigmine adsorbed to the packaging bag after the patch products fabricated in Example 2, Comparative Example 3, and Comparative Example 4 to be described later were stored under a severe condition (60°C) for predetermined periods of time (4 days, 7 days, and 14 days) was measured and evaluated in conformity to the following [Content Measurement Procedure (3)]. The results thus obtained are presented in Table 3.

[Content Measurement Procedure (3)]

<Amount of Drug Remaining in Patch (3)>

After the lapse of predetermined periods of time, the patch was taken out from the patch product, the separator layer was peeled off from the patch, the patch was then immersed in a sealable glass container containing an internal standard solution and tetrahydrofuran for HPLC to dissolve the pressure sensitive adhesive, and a mixed solution of acetonitrile for pharmaceutical/sodium dihydrogen phosphate buffer (pH: 5.8) was then added to the container to prepare a sample solution (patch).
Separately, a standard solution was prepared by using standard rivastigmine through the same operation.
These sample solution and standard solution were analyzed together by a high performance liquid chromatography (HPLC) method. The amount of rivastigmine (drug content in patch) in the sample (patch) was calculated from the ratio of the peak area of rivastigmine to the peak area of the internal standard substance in the standard solution and the sample solution (patch).

<Amount of Drug Adsorbed to Packaging Bag (2)>

The packaging bag of the patch product stored under a severe condition was finely cut and used to prepare a sample solution (packaging bag) in conformity to the following procedure of <Amount of Drug Remaining in Patch (3)>, and the amount of rivastigmine (drug content in packaging bag) in the sample (packaging bag) was calculated by the same procedure using a standard solution.

<Evaluation>

From the results thus obtained, the adsorbed amount of drug (%) was calculated based on the following formula, and the average value of 3 times of measurement was calculated from this value and evaluated as follows.

$$\text{Adsorbed amount of drug (\%)} = [\text{drug content in packaging bag}/(\text{drug content}$$

$$\text{in packaging bag} + \text{drug content in patch})] \times 100$$

Judgment ○: amount of drug (rivastigmine) adsorbed to packaging bag is less than 1%
Judgment ×: amount of drug (rivastigmine) adsorbed to packaging bag is 1% or more

[Example 2]

DURO-TAK (registered trademark) 87-2516 (containing a hydroxyl group, Henkel) as an acrylic pressure sensitive adhesive and ethyl acetate were mixed together at room temperature and the respective blending proportions of 40 mass% and 60 mass% to prepare an acrylic pressure sensitive adhesive solution (the numerical value (%) is based on the total mass of the pressure sensitive adhesive solution). Subsequently, this solution was coated on a PET film (FILMBYNA (registered trademark) 75E-0010 No. 23, manufactured by FUJIMORI KOGYO CO., LTD.) which had a thickness of 75 μm and was subjected to silicone release treatment as a separator layer so as to have a thickness after drying of 80 μm and dried at from 60°C to 100°C, thereby forming a pressure sensitive adhesive layer.
Rivastigmine and EUDRAGIT (registered trademark) EPO (Evonik Degussa) were respectively weighed so as to be 90 mass% and 10 mass%, these were put in a glass bottle and dissolved at room temperature, thereby obtaining a rivastigmine solution (the numerical value (%) is based on the total mass of the rivastigmine solution). A rivastigmine-containing layer was forming by coating the rivastigmine solution thus obtained on a PET film (EMBLET (registered trademark) S grade, manufactured by UNITIKA LTD.) having a thickness of 25 $\mu$m as a support to have a thickness of 20 $\mu$m.

The pressure sensitive adhesive layer formed on the silicone release-treated PET film and the rivastigmine-containing layer formed on the PET film were allowed to face each other, pasted, and cut, thereby fabricating a rectangular patch having two layers of a pressure sensitive adhesive layer containing an acrylic pressure sensitive adhesive and a drug-storing layer containing rivastigmine. The separator layer was larger than the size of patch by about 1.5-fold and had a rectangular shape (R is provided to four corners) with rounded corners, and the patch was disposed in the center of the separator layer.

The patch thus fabricated was enclosed in a packaging bag obtained by allowing laminates (see below) having a layer configuration in which a COC film on the innermost layer side, an aluminum film, and a polyester film were laminated in this order to face each other and heat-sealing three sides of the facing laminates. Subsequently, the rest one side of the packaging bag was sealed by heat sealing in a state in which the interior of the packaging bag was purged with nitrogen by using a vacuum gas packaging machine and the nitrogen inside the packaging bag was extruded (discharged) by allowing the packaging bag to pass between two rolls, thereby fabricating a rectangular patch product (product size: 60 mm x 60 mm, four sides were heat-sealed, sealed width: 5 mm).

The oxygen concentration was 10 vol% when the oxygen concentration inside the packaging bag was measured by using an oximeter.

Incidentally, the details of the laminate used in Example 2 are as follows.

Laminate configuration: COC film/adhesive/aluminum film (AL)/adhesive/polyester film (PET)
COC film: cyclic olefin copolymer, thickness: 30 $\mu$m
Polyester film: polyethylene terephthalate film, thickness: 12 $\mu$m
Aluminum film: aluminum foil, thickness: 7 $\mu$m
Adhesive: polyurethane for adhesive

[Comparative Example 3]

A patch product was fabricate by the same method as in Example 2 except that the innermost layer of the packaging bag was changed to a polyester film (PET, thickness: 12 $\mu$m).

[Comparative Example 4]

A patch product was fabricated by the same method as in Example 2 except that the innermost layer of the packaging bag was changed to a polyethylene film (thickness: 30 $\mu$m).
[Table 3]

Table 3

| | | After 4 days | After 7 days | After 14 days |
|---|---|---|---|---|
| Amount of drug adsorbed to packaging bag [stored at 60°C] | Example 2 | ○ | ○ | ○ |
| | Comparative Example 3 | × | × | - |
| | Comparative Example 4 | × | × | - |

From the results in Table 3, it can be seen that a result that the temporal stability of rivastigmine is significantly excellent as the adsorption of drug to the packaging bag is minor (the adsorbed amount of drug is less than 1 %) even after the lapse of 14 days under a severe condition of 60°C has been obtained in Example 2 in which a rivastigmine-containing patch is enclosed in a packaging bag composed of a laminate having a COC film layer as the innermost layer.

On the other hand, a poor result that temporal stability of rivastigmine is lacking as the amount of drug adsorbed to the packaging bag already exceeds 1% after the lapse of 4 days and a large amount of the drug is adsorbed to the packaging bag has been obtained in Comparative Example 3 in which the innermost layer is a polyester film and Comparative Example 4 in which the innermost layer is a polyethylene film.

# EP 3 238 689 A1

**Claims**

1. A patch product comprising:

   a packaging bag; and
   a patch which contains a drug and is housed in the packaging bag, wherein
   the packaging bag is formed of a laminate composed of at least three or more layers including a cyclic polyolefin film, wherein
   the laminate has a laminate configuration having a heat sealable cyclic polyolefin film as an innermost layer of the packaging bag, an oxygen blocking layer as a layer on an outer side of the cyclic polyolefin film, and an outer layer as a layer on an outer side of the oxygen blocking layer disposed, respectively.

2. The patch product according to claim 1, wherein
   the patch includes:

   a support, drug-containing layer which contains a drug and is provided on at least one surface of the support, and a pressure sensitive adhesive layer provided on the drug-containing layer, or
   a support, a pressure sensitive adhesive layer provided on at least one surface of the support, and a drug-containing layer which contains a drug and is provided on the pressure sensitive adhesive layer, or
   a support and a drug-containing pressure sensitive adhesive layer provided on at least one surface of the support.

3. The patch product according to claim 2, wherein
   the support is a fabric selected from the group consisting of a nonwoven fabric, a woven fabric, and a knitted fabric.

4. The patch product according to claim 2 or 3, wherein
   the support includes at least one kind of material selected from the group consisting of polyester and polyolefin.

5. The patch product according to any one of claims 2 to 4, wherein the drug is lidocaine.

6. The patch product according to any one of claims 2 to 4, wherein the drug is a drug which volatilizes at room temperature.

7. The patch product according to claim 6, wherein the drug is rivastigmine.

8. The patch product according to claim 5, wherein
   a content of a drug contained in a drug-containing layer is from 0.1 mass% to 50 mass% based on a total mass of the drug-containing layer and a pressure sensitive adhesive layer, or
   a content of a drug contained in a drug-containing pressure sensitive adhesive layer is from 0.1 mass% to 50 mass% based on a total mass of the drug-containing pressure sensitive adhesive layer in the patch.

9. The patch product according to claim 7, wherein
   a content of a drug contained in a drug-containing layer is from 10 mass% to 40 mass% based on a total mass of the drug-containing layer and a pressure sensitive adhesive layer, or
   a content of a drug contained in a drug-containing pressure sensitive adhesive layer is from 10 mass% to 40 mass% based on a total mass of the drug-containing pressure sensitive adhesive layer in the patch.

10. The patch product according to claim 9, wherein
    the pressure sensitive adhesive layer or the drug-containing pressure sensitive adhesive layer contains an acrylic pressure sensitive adhesive.

11. The patch product according to any one of claims 1 to 10, wherein a residual oxygen concentration in the packaging bag is 10 vol% or less.

12. A packaging body for a patch containing lidocaine, wherein
    the packaging body is formed of a laminate composed of at least three or more layers including a cyclic polyolefin film, wherein
    the laminate has a laminate configuration having a heat sealable cyclic polyolefin film as an innermost layer of the packaging body, an oxygen blocking layer as a layer on an outer side of the cyclic polyolefin film, and an outer layer as a layer on an outer side of the oxygen blocking layer disposed, respectively.

13. A packaging body for a patch containing rivastigmine, wherein
the packaging body is formed of a laminate composed of at least three or more layers including a cyclic polyolefin film, wherein
the laminate has a laminate configuration having a heat sealable cyclic polyolefin film as an innermost layer of the packaging body, an oxygen blocking layer as a layer on an outer side of the cyclic polyolefin film, and an outer layer as a layer on an outer side of the oxygen blocking layer disposed, respectively.

14. The packaging body according to claim 12 or claim 13, wherein a residual oxygen concentration in the packaging body is 10 vol% or less.

15. A method of manufacturing the patch product according to any one of claims 1 to 11, the method comprising:

    a step of preparing a laminate which includes a film of cyclic polyolefin and has a heat sealable layer as a surface layer;
    a step of fabricating a bag-like article provided with an opening by superposing the two laminates such that the heat sealable layers are face each other and heat-sealing the superposed laminates;
    a step of loading a patch in the bag-like article through the opening; and
    a step of fabricating a packaging bag with patch by sealing the opening of the bag-like article by heat sealing.

16. The manufacturing method according to claim 15, further comprising:

    a step of purging an interior of the bag-like article with nitrogen and/or loading an oxygen absorbent in the bag-like article through the opening; and
    a step of discharging a gas inside the bag-like article to an outside to an extent that the two laminates tightly sandwich the patch by roll pass, pressing, or suction degassing
    before the step of fabricating a packaging bag with patch by sealing the opening of the bag-like article by heat sealing.

17. A method of manufacturing the patch product according to any one of claims 1 to 11, the method comprising:

    a step of preparing a laminate which includes a film of cyclic polyolefin and has a heat sealable layer as a surface layer;
    a step of disposing a patch on the heat sealable layer of one laminate;
    a step of disposing the other laminate on the one laminate such that the heat sealable layers of the two laminates face each other via the patch;
    a step of forming an opening by heat-sealing a greater part of the two heat sealable layers facing each other but not heat-sealing a part of the two heat sealable layers; and
    a step of fabricating a packaging bag with patch by heat-sealing the opening.

18. The manufacturing method according to claim 17, further comprising the following steps before fabricating a packaging bag with patch by heat-sealing the opening:

    a step of purging an atmosphere in a vicinity of the patch to be formed by the two laminates with nitrogen and/or loading an oxygen absorbent in a vicinity of the patch; and
    a step of discharging a gas in a vicinity of the patch to an outside through the opening to an extent that the two laminates tightly sandwich the patch by roll pass, pressing, or suction degassing.

## INTERNATIONAL SEARCH REPORT

International application No.

PCT/JP2015/082476

### A. CLASSIFICATION OF SUBJECT MATTER

*A61J1/00*(2006.01)i, *A61K9/70*(2006.01)i, *A61K31/167*(2006.01)i, *A61K31/27*
(2006.01)i, *A61K45/00*(2006.01)i, *A61K47/06*(2006.01)i, *A61K47/14*(2006.01)i,
*A61K47/32*(2006.01)i, *A61K47/34*(2006.01)i, *A61P23/02*(2006.01)i

According to International Patent Classification (IPC) or to both national classification and IPC

### B. FIELDS SEARCHED

Minimum documentation searched (classification system followed by classification symbols)
A61J1/00, A61K9/70, A61K31/167, A61K31/27, A61K45/00, A61K47/06,
A61K47/14, A61K47/32, A61K47/34, A61P23/02

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)
CAplus/REGISTRY(STN)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 2012-086876 A (Dainippon Printing Co.,<br>Ltd.),<br>10 May 2012 (10.05.2012),<br>paragraphs [0009], [0024] to [0025], [0032],<br>[0034] to [0035]; fig. 2<br>(Family: none) | 1<br>2-18 |
| Y | WO 2008/044336 A1 (Hisamitsu Pharmaceutical<br>Co., Inc.),<br>17 April 2008 (17.04.2008),<br>paragraphs [0016], [0019], [0024], [0028]; fig.<br>1<br>& JP 5243254 B | 2-14 |

[X] Further documents are listed in the continuation of Box C.        [ ] See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04 December 2015 (04.12.15) | 15 December 2015 (15.12.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (July 2009)

**INTERNATIONAL SEARCH REPORT**

International application No.

PCT/JP2015/082476

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2013/027840 A1 (Nipro Patch Co., Ltd.), 28 February 2013 (28.02.2013), paragraphs [0017], [0020] & US 2014/0302118 A1 paragraphs [0022], [0025] & EP 2749279 A1 & CA 2846121 A & KR 10-2014-0054248 A & CN 103930102 A | 8,11 |
| Y | WO 2014/34939 A1 (Nipro Patch Co., Ltd.), 06 March 2014 (06.03.2014), paragraphs [0016], [0019] to [0020], [0047] & US 2015/0209302 A1 paragraphs [0029], [0034] to [0035], [0068] & EP 2893927 A1 | 9,11,16-18 |
| Y | JP 2012-051875 A (Hisamitsu Pharmaceutical Co., Inc.), 15 March 2012 (15.03.2012), claims 6 to 8; paragraphs [0024], [0035] to [0036] & US 2012/0031047 A1 claims 6 to 8; paragraphs [0026], [0039] to [0040] | 11,14,16-18 |
| Y | WO 2005/072716 A1 (Hisamitsu Pharmaceutical Co., Inc.), 11 August 2005 (11.08.2005), paragraph [0062] & JP 4989892 B & US 2007/0158227 A1 paragraph [0060] | 15-18 |
| Y | JP 08-081363 A (Sekisui Chemical Co., Ltd.), 26 March 1996 (26.03.1996), paragraph [0017] (Family: none) | 16-18 |

Form PCT/ISA/210 (continuation of second sheet) (July 2009)

**REFERENCES CITED IN THE DESCRIPTION**

**Patent documents cited in the description**

- JP 2001009985 A
- JP 3575616 B
- JP 2010280403 A
- JP 3115625 B
- WO 2011027786 A
- JP 6145051 A
- JP 2004292379 A
- JP 2002500178 W
- JP 2009517468 W
- WO 2011076621 A
- US 20110066120 A
- EP 2172194 B